# EUROPEAN PATENT APPLICATION

(11) **EP 4 763 186 A1**
(43) Date of publication of application: **24.06.2026**
(21) Application number: 24383446.2
(22) Date of filing: 21.12.2024
(51) Int. Cl.: A61K 8/27, A61K 9/00, A61K 9/06, A61K 9/08, A61K 9/12, A61K 9/127, A61K 9/1277, A61K 9/19, A61K 33/30, A61K 36/738, A61P 17/02, A61K 36/23

(54) **LIPOSOMAL COMPOSITION**

(71) Applicant: Botet Carreras, Adrià, 08026 Barcelona (ES)
(72) Inventor: Botet Carreras, Adrià, 08026 Barcelona (ES); Borrell Hernández, Jordi, 08800 Vilanova i la Geltrú (ES); Domenech Cabrera, Oscar, 17230 Palamos (ES)

(57) **Abstract**

Liposomal composition

It refers to a to a liposomal composition comprising a *Rosa eglanteria* extract, a *Centella asiatica* extract, zinc oxide, and a buffer. It refers also to the process for preparing said composition, to a pharmaceutical composition comprising it, to its use in medicine and cosmetics, in particular for treating wounds and scars

RESUMEN

Composición liposomal

La presente invención se refiere a una composición liposomal compuesta por un extracto de *Rosa eglanteria,* un extracto de *Centella asiática,* óxido de zinc y un tampón. Se refiere también al proceso de preparación de dicha composición, a una composición farmacéutica que la comprende, y a su uso en medicina y en cosmética, en particular para el tratamiento de heridas y cicatrices.

## Description

### FIELD OF THE INVENTION

The present invention relates to the field of compositions suitable for treating wounds and scars, in particular, those that are permanently exposed.

### BACKGROUND ART

Highly complex and chronic wounds continue to be a major problem today, taking into account the increase in life expectancy, people live longer with chronic diseases, which in many cases become risk factors in the healing process of these wounds. The treatment of chronic wounds is an issue of great relevance for the health systems, as it leads to a decrease in the patient's quality of life, frequent care, bad smell, pain, loss of physical mobility, as well as high expense in material and personal resources. In consequence, it is essential to provide a correct therapeutic measure that facilitates and accelerates its resolution. The efficacy and effectiveness of the measures applied can be assessed with parameters such as: time to complete healing, changes in the volume of the lesion, changes in the depth of the wound, changes in the area, complication rate, and hospitalization rate.

Chronic wounds types are, for example, pressure ulcers, venous and arterial ulcers, and diabetic foot ulcers.

Pressure ulcers, also known as pressure sores, bed sores, or dependency-related injuries, are areas of localized deterioration in the skin and underlying tissue with loss of skin substance. They are produced by prolonged pressure. The pressure causes tissue crushing that obstructs blood flow from the skin and underlying tissues, and promotes the appearance of tissue ischemia and necrosis, leading to a pressure ulcer.

Venous ulcers are lesions with loss of skin integrity, due to the incompetence of the venous system. The lesion is located on the skin previously damaged by secondary dermatitis. Venous ulcers represent the final stage of chronic venous disorder.

Arterial ulcers are lesions or wounds caused by a decrease in blood perfusion and the result of a critical deficit in the partial pressure of oxygen in the distal tissues. The underlying pathology is chronic peripheral arterial obstruction secondary to arteriosclerosis, which is characterized by the formation of atheromatous plaques that obstruct the arterial lumen causing a reduction in blood flow to the distal tissues of the arterial tree. This type of ulcer appears in advanced stages of chronic ischemia.

Diabetic foot ulcers are defined as the presence of ulceration, infection and/or gangrene in the foot associated with diabetic neuropathy and different degrees of peripheral vascular disease as a result of the complex interaction of different factors induced by maintained hyperglycaemia.

Chronic wound management includes the initial debridement of the necrotic tissue, before using one of the many treatment strategies. The known methods of accelerating chronic wound healing include: pressure relief, hydrotherapy, ultrasonic treatment, electrical stimulation, hyperbaric oxygenation, vacuum assisted closure therapy, skin grafts and artificial skin replacements as well as methods from the tissue engineering field, surgical venous rehabilitation, gene therapy, use of protease regulating wound dressings, use of growth factor-loaded gels. However, said methods have some disadvantages and drawbacks. Namely, some of them are very expensive and for that reason not commonly available, some of them demand regimens which are very difficult or painful for a patient, and quite frequently they are simply not sufficiently effective since chronic wounds are characterized by a very high recurrence rate.

Another approach for improving chronic wound healing is the use of natural materials, as disclosed in Moses et al., Evidence for Natural Products as Alternative Wound-Healing Therapies, biomolecules, 2023, 13, 444. This review discloses specific clinical trials of bioactive small molecules from natural sources for wound healing, as well a selection of bioactive small molecules from natural sources with promising wound-healing potential. Among the natural sources, the following are mentioned: an extract from the French maritime pine *Pinus pinaster* Aiton; an hydroglycolic extract of *Calendula officinalis;* a polyherbal cream containing a variety of plant products: *Glycyrrhiza glabra, Musa paradisiaca, Curcuma longa, Pandanus odoratissimus, Aloe vera,* and *Cocos nucifera;* a combination of *Aloe vera* and *Plantago major,* a cream formulation of *Ageratina pichinchensis;* an extract of *Symphytum uplandicum;* a salve of *Picea abies;* a formulation of *Triticum vulgare;* cortex extract of *Mimosa teniuflora;* alcohol extracts of *Calendula officinalis, Symphytum officinale, Achillea millefolium,* and *Salvia officinalis;* gel of *Aesculus hippocastanum, Melilotus officinalis, Rosmarinus,* and *Lavandula;* extracts of garlic, St John's wort and calendula; cream of *Aloe vera* and olive oil; Manuka honey from bees utilising the flower *Leptospermum scoparium*; an exudate of *Lepiniopsis ternatensis;* an exudate of *Cypholophus macrocephalus;* and sap from the Beser variety of *Phoenix dactylifera.*

In international patent application WO-A-2009/053850 it is disclosed an herbal extract from *Mellilotus* sp. (*Mellilotus officinalis*)*,* optionally comprising selenium and/or urea and/or fructose and/or phosphoglycerol, which is useful in the treatment of chronic wounds, in particular associated with states in which the normal wound repair ability is weakened, and preferably diabetic foot ulcers and/or bed sores.

In German patent application DE-A-19952190 it is disclosed the use of plant starch in wound healing.

Thus, in spite of the different proposals for treating wounds disclosed in the prior art, there is a need for effective compositions, in particular for treating wounds and scars that are permanently exposed.

### SUMMARY OF INVENTION

An aspect of the invention is a liposomal composition.

Another aspect of the present invention is the process for preparing said composition.

Another aspect of the invention is a pharmaceutical composition comprising the liposomal composition.

Another aspect of the invention is the liposomal composition and the pharmaceutical composition for use in medicine.

Another aspect of the invention is the cosmetic use of the liposomal composition.

### FIGURES

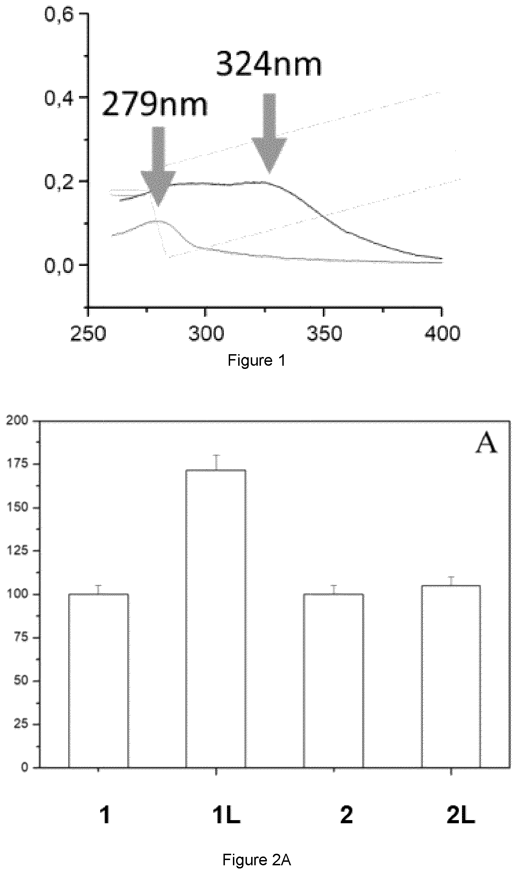

In Figure 1, spectrophotometric UV curves of *Rosa eglanteria* and *Centella asiatica* extracts are shown. The peak at 279 nm of *Rosa eglanteria* is attributed to gallic acid. The peak at 324 nm corresponds to a broad maximum of *Centella asiatica.* Those peaks were used in the HPLC UV-Vis detector. The wavelength expressed in nm is shown in abscises, and the absorbance expressed in UA is shown in the ordinates.

In Figure 2A are shown the results of permeation studies of free active extracts in the solution and in the presence of liposomes, when the receiving medium is Transcutol^{®}, wherein in abscises, 1 corresponds to *Rosa eglanteria* extract, 1L corresponds to *Rosa eglanteria* extract encapsulated in liposomes, 2 corresponds to *Centella asiatica* extract, and 2L corresponds to *Centella asiatica* extract encapsulated in liposomes. In ordinates it is represented the amount of extract in the skin expressed in µg/cm².

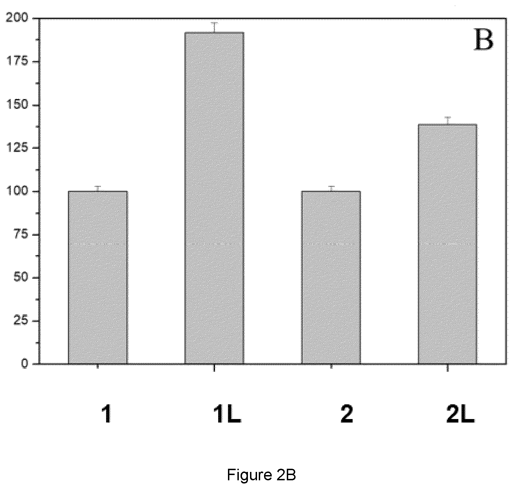

In Figure 2B are shown the results of permeation studies of free active extracts in the solution and in the presence of liposomes, when the receiving medium is a buffer water-based salt solution (10 mM Tris·HCl, 150 mM NaCl buffer, pH 7.4), wherein in abscises, 1 corresponds to *Rosa eglanteria* extract, 1L corresponds to *Rosa eglanteria* extract encapsulated in liposomes, 2 corresponds to *Centella asiatica* extract, and 2L corresponds to *Centella asiatica* extract encapsulated in liposomes. In ordinates it is represented the amount of extract in the skin expressed in µg/cm².

### DETAILED DESCRIPTION OF THE INVENTION

The present invention relates to a liposomal composition comprising:
a) liposomes,
b) a *Rosa eglanteria* extract,
c) a *Centella asiatica* extract,
d) zinc oxide, and
e) a buffer,
wherein at least 50 wt%, preferably at least 55 wt%, preferably at least 60 wt%, preferably at least 65 wt%, preferably at least 70 wt%, more preferably at least 80 wt%, and yet more preferably at least about 84 wt% of said extracts are encapsulated in the liposomes.

The inventors of the present invention have developed a liposomal composition, suitable for treating wounds and scares, in particular, those that are permanently exposed, such as derived from lupus, scar regeneration, or treating recurrent wounds in patients who spent long periods in bed. Said liposomal composition comprises the actives partially encapsulated in liposomes, and surprisingly provides a double release through the skin, with an immediate delivery of the active ingredients followed by a sustained release thereof from the liposomes.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

### Liposomal composition

In the context of the present invention the term liposomal composition means a composition that comprises liposomes.

As used in this description, "liposome" is used to describe oligo-lamellar lipid vesicles comprising one or more natural or synthetic lipid bi-layers surrounding an internal aqueous phase.

Liposomes are manufactured microscopic, hollow spherical vesicles composed of a lipid bilayer. When loaded with an active component, such as pharmaceuticals, cosmetic actives and/or dietary supplements, liposomes are a very effective method of active delivery. Liposomes are commonly used in cosmetic formulations for improving dermal penetration of active principles.

As is well known in the art, liposomes are spherical vesicles with sizes generally in the range between about 60 nm up to 1µm, depending on the method of preparation. Liposomes contain hydrophilic cores in which hydrophilic actives may be encapsulated, while hydrophobic actives are incorporated in the bilayer, so liposomes are suitable carriers for both hydrophilic and lipophilic actives, as disclosed in, for example, Knoth et al., Nanocarrier-Based Formulations: Production and Cosmeceutic Applications, in: Cosmetic Formulation. Principles and Practice, Benson H.A.E., Roberts M.S., Rodrigues Leite-Silva V. and Walters K.A., editors, CRC Press, 2019.

The lipids that may be used for preparing liposomes include natural and/or synthetic phospholipids, such as phosphatidylcholine (also known as lecithin), phosphatidylethanolamine, phosphatidylglycerol, phosphatidylserine, or phosphatidylinositol.

In an embodiment, the liposomal composition comprises liposomes, which comprise phosphatidylcholine.

Liposome bilayers may also contain other lipids such as cholesterol or hydrophilic polymer conjugated lipids.

In an embodiment, the liposomal composition comprises liposomes, which comprise a combination of phosphatidylcholine and cholesterol. In an embodiment, the liposomes comprise between 70:30 molar ratio and 95:5 molar ratio, preferably about 85:15 molar ratio of phosphatidylcholine and cholesterol.

In a preferred embodiment, the liposomal composition comprises liposomes, which consists of phosphatidylcholine. In this embodiment, the content of phosphatidylcholine in the liposomal composition of the invention is comprised between 5 mg and 15 mg, preferably between 6 mg and 14 mg, preferably between 7 mg and 13 mg, more preferably between 8 mg and 12 mg, and yet more preferably about 10 mg per mL of the liposomal composition.

In a preferred embodiment, the liposomal composition comprises liposomes, which consists of a combination of phosphatidylcholine and cholesterol. In this embodiment, the content of phosphatidylcholine in the liposomal composition of the invention is comprised between 5 mg and 15 mg, preferably between 6 mg and 14 mg, preferably between 7 mg and 13 mg, more preferably between 8 mg and 12 mg, and yet more preferably about 9.8 mg per mL of the liposomal composition, and the content of cholesterol is comprised between 0.1 mg and 15 mg, preferably between 0.3 mg and 10 mg, preferably between 0.5 mg and 5 mg, more preferably between 0.7 mg and 1 mg, and yet more preferably about 0.88 mg per mL of the liposomal composition.

In the liposomal composition of the invention at least 50 wt%, preferably at least 55 wt%, preferably at least 60 wt%, preferably at least 65 wt%, preferably at least 70 wt%, more preferably at least 80 wt%, and yet more preferably at least 84 wt% of said extracts are encapsulated in liposomes.

In an embodiment, the liposomal composition comprise the liposomes, which comprise between 75 wt% and 95 wt%, preferably between 80 wt% and 90 wt%, more preferably about 85 wt% of *Rosa eglanteria* extract and 5 wt% and 25 wt%, preferably between 10 wt% and 20 wt%, more preferably about 15 wt% of *Centella asiatica* extract on the total extract content encapsulated in the liposomes..

In order to establish the lipid/drug ratio of the liposomal composition it is quantified the amount of extracts contained in the liposomes.

The quantification of the amount of extracts encapsulated in the liposomes of the liposomal composition may be performed, for example, using the spin column method as disclosed in D. Sterchi, Molecular Pathology - In Situ Hybridization, in book Theory and Practice of Histological Techniques, 2008, pages 546 - 547. In that method, spin columns provide a straightforward way of separating molecules by molecular weight. This is achieved by centrifuging the solution through a column, e.g., a syringe, of packed crosslinked dextran, such as, for example, Sephadex^{®} G-50, Sephacryl^{®} S-400, or Probe Quant^{®} G-50, in which the low molecular weight molecules are retained but the larger molecular weight molecules are excluded and, therefore, are recovered in the eluent.

The method comprises:
1) filtering the liposomal composition using the spin column method through a column made of a crosslinked dextran gel filled in a syringe,
2) collecting the loaded liposomes from the flow leaving the syringe, and
3) analyzing by HPLCS the loaded liposomes to determine their extract content.

Using this crosslinked dextran gel, the free extracts are retained thereon, because the gel is able to separate molecules by molecular weight. This method is suitable to calculate the extract/lipid ratio, which provides information on the yield of the encapsulation process.

In an embodiment, the crosslinked dextran gel is Sephadex^{®} G-50, and the filtering is performed using a centrifuge at 1500 rpm, 5 min, at 4 °C.

The measurement of the polydispersity index of the liposomal composition, which indicates the broadness of the particle size distribution measured by the diffraction of the liposomes by means of a laser both in absence and in presence of ZnO, the presence of ZnO surprisingly reduced the polydispersity of the liposomal composition from 0.697 to 0.273 meaning a more stable and regular sizing of the generated liposomes.

### Rosa eglanteria extract

*Rosa eglanteria,* also known as *Rosa rubiginosa,* it is a shrub of the Rosaceae family that can exceed two meters in height. It has curved stems covered with violet stingers. Its flowers measure from 3 to 6 centimetres in diameter and form racemose-shaped corymb-shaped inflorescences of 2 to 7 or 15 units.

The process for preparing the extract of *Rosa eglanteria* may be carried out following substantially the disclosure of Sallustio et al., Extraction, Encapsulation into Lipid Vesicular Systems, and Biological Activity of Rosa canina L. Bioactive Compounds for Dermocosmetic Use, molecules, 2022, 27, 3025.

The extract of *Rosa eglanteria* is obtained from the whole fruit thereof.

In a preferred embodiment, the extract of *Rosa eglanteria* is a hydroalcoholic extract.

In an embodiment, the *Rosa eglanteria* hydroalcoholic extract is freeze-dried.

In a preferred embodiment, a freeze-dried hydroalcoholic extract of *Rosa eglanteria* is used.

Usually the hydroalcoholic extraction of *Rosa eglanteria* whole fruit yields between 0.1 g and 0.5 g, preferably between 0.15 g and 0.45 g, more preferably between 0.2 g to 0.4 g, and yet more preferably between 0.25 g and 0.35 g of freeze-dried solids per gram of *Rosa eglanteria* starting material.

The content of freeze-dried *Rosa eglanteria* hydroalcoholic extract in the liposomal composition of the invention is comprised between 1 mg and 20 mg, preferably between 2 mg and 18 mg, preferably between 4 and 16 mg, preferably between 6 mg and 14 mg, more preferably between 8 mg and 12 mg, and yet more preferably about 10 mg per mL of the liposomal composition.

### Centella asiatica extract

*Centella asiatica,* also known as Indian pennywort or gotu kola, it is herbaceous, perennial plant in the flowering plant family Apiaceae. It is native to tropical regions of Africa, Asia, Australia, and islands in the western Pacific Ocean.

The process for preparing the extract of *Centella asiatica* may be carried out following substantially the disclosure of Sallustio *et al., op. cit.*

Hydroalcoholic extracts of *Centella asiatica* are commercially available, for example, through different companies Laboratory Guinama (Asiatic Pennywort Fluid Extract), Laboratory Cento Fiori (Centella, hydro alcoholic extract), Laboratory Phybio (Centella asiatica Mother Tincture), Laboratory Naturopath Herbals (Gotu Kola Liquid Extract Tincture), or Laboratory PromoPharma S.p.A (Centella asiatica Soluzione idroalcoholica).

The extract of *Centella asiatica* is obtained from the aerial parts thereof.

In a preferred embodiment, the extract of *Centella asiatica* is a hydroalcoholic extract.

In an embodiment, the *Centella asiatica* hydroalcoholic extract is freeze-dried.

In a preferred embodiment, a freeze-dried hydroalcoholic extract of *Centella asiatica* is used.

The content of freeze-dried *Centella asiatica* hydroalcoholic extract in the liposomal composition of the invention is comprised between 1 mg and 20 mg, preferably between 2 mg and 18 mg, preferably between 4 and 16 mg, preferably between 6 mg and 14 mg, more preferably between 8 mg and 12 mg, and yet more preferably about 10 mg/mL.

### Zinc oxide

The liposomal composition comprises zinc oxide.

Zinc oxide is an inorganic compound with the formula ZnO. It is a white powder which is insoluble in water. ZnO is used as an additive in numerous materials and products including cosmetics, food supplements, rubbers, plastics, ceramics, glass, cement, lubricants, paints, sunscreens, ointments, adhesives, sealants, pigments, foods, batteries, ferrites, fire retardants, semiconductors, and first-aid tapes. Although it occurs naturally as the mineral zincite, most zinc oxide is produced synthetically.

The zinc oxide is present in the liposomal composition is dissolved state. In an embodiment, ZnO is present at saturation concentration in the aqueous phase, at about 0.0004 wt%.

In an embodiment, the content of zinc oxide is about 3 µg per mL of the liposomal composition.

### The buffer

The liposomal composition comprises a buffer.

In an embodiment, the buffer comprises Tris-HCl and sodium chloride, NaCl, adjusted at a pH of about 7.4.

Tris is the common abbreviation of tris(hydroxymethyl)aminomethane.

In an embodiment the buffer comprises 10 mM Tris-HCl and 150 mM NaCl.

The buffer is the aqueous vehicle of the liposomal formulation.

### Process for preparing the liposomal composition

An aspect of the invention is a process for preparing the liposomal composition.

Liposomes can be prepared by any conventional method as disclosed in, for example, Lombardo et al., Methods of Liposomes Preparation: Formation and Control Factors of Versatile Nanocarriers for Biomedical and Nanomedicine Application, pharmaceutics, 2022, 14, 543.

The accurate selection of liposome preparation method depends on the physicochemical characteristics of the material to be entrapped, choice of liposomal ingredients, nature of the medium in which the lipid vesicles are to be dispersed, effective concentration of the entrapped substance, optimum size and shelf life of the vesicle, and batch-to-batch reproducibility.

In the context of the invention, the process for preparing the liposomal composition is based on the Thin-film hydration method (Bangham method) and it comprises the following steps:
1) dissolving the lipid in an organic solvent, in a vessel,
2) evaporating the solvent of the solution obtained in step 1) to yield a film of the lipid on the walls of the vessel,
3) redispersing the film obtained in step 2) with a buffer containing the extract of *Rosa Englanteria* and/or the extract of *Centella asiatica,* and zinc oxide at saturation, and
4) sonicating the dispersion obtained in step 3) to form liposomes.

In an embodiment, the buffer contains both extracts.

In an embodiment, the dispersion in Step 3) is sonicated at least 5 times of freeze-thaw cycles crossing the transition temperature of the lipids used to prepare the liposomes.

In an embodiment, the liposomal composition is freeze-dried, and the obtained powder may be used for the preparation of a pharmaceutical composition.

In an embodiment, the liposomal is freeze dried adding a cryoprotectant. The cryoprotectant may be selected from, for example, cellobiose, glucose, lactose, mannitol, sucrose, trehalose, and mixtures thereof. In a preferred embodiment, the cryoprotectant is selected from lactose and/or cellobiose. In a preferred embodiment, the content of cryoprotectant is 5 wt% over the weight of the aqueous solution obtained in step 4).

In an embodiment, the liposomes of the extracts are prepared separately. Once each liposomal composition is prepared, the process may include any one of the following steps: mixing both liposomal compositions and using the mixture for the preparation of a pharmaceutical composition, mixing both liposomal compositions and freeze-drying the mixture to use for the preparation of a pharmaceutical composition, or freeze-drying each liposomal composition independently and mixing the resulting powder for the preparation of a pharmaceutical composition.

In an embodiment, the solvent for dissolving the lipid is selected from chloroform, dichloromethane, diethyl ether, methanol, ethanol, isopropanol, and mixtures thereof.

In a preferred embodiment, the solvent is a mixture of chloroform and methanol, more preferably in a ratio of 2:1 (v/v).

### Pharmaceutical composition

An aspect of the invention is a pharmaceutical composition, which comprises the liposomal composition of the invention.

The liposomal composition can be formulated in different final forms, for example: extemporaneous spray, extemporaneous drops, gel comprising between 1 wt% and 5 wt%, preferably about 3 wt% of carboxymethylcellulose (CMC), hydrocolloid patch comprising between 1 wt% and 5 wt%, preferably about 2 wt% of carboxymethylcellulose (CMC) and between 0.5 wt% and 3 wt%, preferably about 1.5 wt%, of glycerol.

### Use of the liposomal composition

An aspect of the invention is the liposomal composition and the pharmaceutical composition for use in medicine.

In an embodiment, the liposomal composition and the pharmaceutical composition is for use in the treatment of wounds and scares, in particular, those that are permanently exposed. The use comprises the step of topically applying a therapeutically effective amount of the liposomal composition or the pharmaceutical composition to the wound or to the scare of a subject in need thereof.

The permeation *ex-vivo* of the liposomal composition of the invention may be tested in human skin using Franz cells of vertical diffusion in the presence of a lipophilic medium (Transcutol^{®}, diethylene glycol ethyl ether) or in the presence of an aqueous buffer as hydrophilic receiving media.

When the receiving medium is Transcutol^{®} the concentration of *Rosa eglanteria* extract in the skin after 24 hours increases by 75% when said extract is encapsulated within liposomes compared to the value when it was free in the medium. Using a buffer solution as a receiving medium instead of Transcutol^{®}, the values obtained with *Rosa eglanteria* extract were very similar to those obtained from Transcutol^{®}, but the values of retained *Centella asiatica* extract in the skin were increased, obtaining values approximately 40% higher when the molecule was in the presence of liposomes than when it was free in the medium. These results are indicative that the active molecules analysed present in *Centella asiatica* do not have much affinity for lipophilic phases and that they prefer to diffuse towards more polar regions.

The present invention may be defined according to the following embodiments:
1.- A liposomal composition comprising:
   a) liposomes,
   b) a *Rosa eglanteria* extract,
   c) a *Centella asiatica* extract,
   d) zinc oxide, and
   e) a buffer,
   f) wherein at least 50 wt%, preferably at least 55 wt%, preferably at least 60 wt%, preferably at least 65 wt%, preferably at least 70 wt%, more preferably at least 80 wt%, and yet more preferably at least about 84 wt% of said extracts are encapsulated in the liposomes.
2.- The liposomal composition according to embodiment 1, wherein it comprises liposomes, which comprise phosphatidylcholine.
3.- The liposomal composition according to embodiments 1 or 2, wherein the content of phosphatidylcholine in the liposomal composition is comprised between 5 mg and 15 mg, preferably between 6 mg and 14 mg, preferably between 7 mg and 13 mg, more preferably between 8 mg and 12 mg, and yet more preferably about 10 mg per mL of the liposomal composition.
4.- The liposomal composition according to embodiment 2, wherein it comprises liposomes, which comprise a combination of phosphatidylcholine and cholesterol.
5.- The liposomal composition according to embodiment 4, wherein the content of phosphatidylcholine in the liposomal composition of the invention is comprised between 5 mg and 15 mg, preferably between 6 mg and 14 mg, preferably between 7 mg and 13 mg, more preferably between 8 mg and 12 mg, and yet more preferably about 9.8 mg per mL of the liposomal composition, and the content of cholesterol is comprised between 0.1 mg and 15 mg, preferably between 0.3 mg and 10 mg, preferably between 0.5 mg and 5 mg, more preferably between 0.7 mg and 1 mg, and yet more preferably about 0.88 mg per mL of the liposomal composition.
6.- The liposomal composition according to any one of embodiments 1 to 5, wherein the liposomal composition comprise the liposomes, which comprise between 75 wt% and 95 wt%, preferably between 80 wt% and 90 wt%, more preferably about 85 wt% of *Rosa eglanteria* extract and 5 wt% and 25 wt%, preferably between 10 wt% and 20 wt%, more preferably about 15 wt% of *Centella asiatica* extract on the total extract content encapsulated in the liposomes.
7.- The liposomal composition according to any one of embodiments 1 to 6, wherein the extract of *Rosa eglanteria* is obtained from the whole fruit.
8.- The liposomal composition according to any one of embodiments 1 to 7, wherein the extract of *Rosa eglanteria* is a hydroalcoholic extract.
9.- The liposomal composition according to embodiment 8, wherein the *Rosa eglanteria* hydroalcoholic extract is freeze-dried.
10.- The liposomal composition according to embodiment 9, wherein the content of freeze-dried *Rosa eglanteria* hydroalcoholic extract in the liposomal composition of the invention is comprised between 1 mg and 20 mg, preferably between 2 mg and 18 mg, preferably between 4 and 16 mg, preferably between 6 mg and 14 mg, more preferably between 8 mg and 12 mg, and yet more preferably about 10 mg per mL of the liposomal composition.
11.- The liposomal composition according to any one of embodiments 1 to 10, wherein the extract of *Centella asiatica* is obtained from the aerial parts thereof.
12.- The liposomal composition according to any one of embodiments 1 to 11, wherein the extract of *Centella asiatica* is a hydroalcoholic extract.
13.- The liposomal composition according to embodiment 12, wherein the *Centella asiatica* hydroalcoholic extract is freeze-dried.
14.- The liposomal composition according to embodiment 13, wherein the content of freeze-dried *Centella asiatica* hydroalcoholic extract in the liposomal composition of the invention is comprised between 1 mg and 20 mg, preferably between 2 mg and 18 mg, preferably between 4 and 16 mg, preferably between 6 mg and 14 mg, more preferably between 8 mg and 12 mg, and yet more preferably about 10 mg/mL.
15.- The liposomal composition according to any one of embodiments 1 to 14, wherein the content of zinc oxide is about 3 µg per mL of the liposomal composition.
16.- The liposomal composition according to any one of embodiments 1 to 15, wherein the buffer comprises Tris-HCl and sodium chloride, NaCl, adjusted at a pH of about 7.4.
17.- A process for preparing the liposomal composition of any one of embodiments 1 to 16, wherein it comprises:
   1) dissolving the lipid in an organic solvent, in a vessel,
   2) evaporating the solvent of the solution obtained in step 1) to yield a film of the lipid on the walls of the vessel,
   3) redispersing the film obtained in step 2) with a buffer containing the extract of *Rosa eglanteria* and/or the extract of *Centella asiatica,* and zinc oxide at saturation, and
   4) sonicating the dispersion obtained in step 3) to form liposomes.
18.- The process according to embodiment 17, wherein the buffer contains both extracts.
19.- The process according to embodiment 17, wherein the liposomes of the extracts are prepared separately.
20.- The process according to any one of embodiments 17 to 19, wherein the liposomal composition is freeze-dried.
21.- The process according to embodiment 20, wherein the process includes any one of the following steps: mixing both liposomal compositions and using the mixture for the preparation of a pharmaceutical composition, mixing both liposomal compositions and freeze-drying the mixture to use for the preparation of a pharmaceutical composition, or freeze-drying each liposomal composition independently and mixing the resulting powder for the preparation of a pharmaceutical composition.
22.- The process according to any one of embodiments 17 to 21, wherein the solvent for dissolving the lipid is selected from chloroform, dichloromethane, diethyl ether, methanol, ethanol, isopropanol, and mixtures thereof.
23.- The process according to embodiment 22, wherein the solvent is a mixture of chloroform and methanol, preferably in a ratio of 2:1 (v/v).
24.- A pharmaceutical composition, which comprises the liposomal composition according to any one of embodiments 1 to 16.
25.- The pharmaceutical composition according to embodiment 24, wherein the form thereof is selected from extemporaneous spray, extemporaneous drops, gel and hydrocolloid patch.
26.- The pharmaceutical composition according to embodiment 25, wherein the gel comprises between 1 wt% and 5 wt%, preferably about 3 wt% of carboxymethylcellulose (CMC).
27.- The pharmaceutical composition according to embodiment 26, wherein the hydrocolloid patch comprises between 1 wt% and 5 wt%, preferably about 2 wt% of carboxymethylcellulose (CMC) and between 0.5 wt% and 3 wt%, preferably about 1.5 wt%, of glycerol.
28.- The liposomal composition according to any one of embodiments 1 to 16 or the pharmaceutical composition according to any one of embodiments 24 to 27 for use in medicine.
29.- The liposomal composition or the pharmaceutical composition according to embodiment 28 for use in the treatment of wounds and scares.
30.- The liposomal composition for use or the pharmaceutical composition for use according to embodiment 29 in the treatment of wounds and scares that are permanently exposed.
31.- The liposomal composition for use or the pharmaceutical composition for use according to embodiment 29 or 30, wherein the use comprises the step of topically applying a therapeutically effective amount of the liposomal composition to the wound or to the scare of a subject in need thereof.
32.- The cosmetic use of the liposomal composition according to any one of embodiments 1 to 16 or the pharmaceutical composition according to any one of embodiments 24 to 27 for the treatment of wounds and scares.

### EXAMPLES

### HPLC analysis

The extracts were analyzed by HPLC (HP1100, Chemstations, Agilent Technologies, USA). The samples were injected into a C18 reversed-phase column (spherisorb ods2 5µ 15x0.46 cm). Wavelength in the UV-Vis detector was set at 279 nm for *Rosa eglanteria* and 324 nm for *Centella asiatica* from absorption spectra of the extracts (see Figure 1) obtained by spectrophotometry (UV-2410PC, Shimadzu, Japan). A mobile phase composed 40/50 by (H₂O + 0.1% orthophosphoric acid)/acetonitrile. The flow rate was 1 mL/min and the analysis time per sample was 3 min.

### Quantification of the extracts encapsulated in the liposomes

The liposomal composition was filtered through a spin column of Sephadex^{®} G-50 with the help of a centrifuge at 1500 rpm, 5 min, at 4 °C. The free extracts present in the liposomal composition were retained on the column leaving the loaded liposomes, which were injected on the HPLC to determine their extract content using the above disclosed method.

The spin column was prepared with the procedure disclosed in Sterchi, *op. cit*., page 547.

### Preparative example 1: Preparation of Rosa eglanteria extract

The process for preparing the extract of *Rosa eglanteria* was carried out following substantially the disclosure of Sallustio et al., Extraction, Encapsulation into Lipid Vesicular Systems, and Biological Activity of Rosa canina L. Bioactive Compounds for Dermocosmetic Use, molecules, 2022, 27, 3025 or of Um et al., Ultrasound-assisted extraction and antioxidant activity of phenolic and flavonoid compounds and ascorbic acid from rugosa rose (Rosa rugosa Thunb.) fruit, Food Sci. Biotechnol., 27(2), 375-382.

10 g of *Rosa eglanteria* in powder form was extracted with 150 ml of a mixture of ethanol: water 50:50 (v/v) with the aid of an ultrasonic water bath at power and frequency of 345 W and 40 kHz. The temperature was comprised between 30 °C and 40 °C, and the time of 40'.

The liquid fraction was separated from the pulp by means of centrifugation at about 40.000 rpm for 20 minutes. The supernatant was filtered with a 0.45 µm PTFE membrane filter.

The liquid fraction obtained after centrifugation was subsequently concentrated, and the concentrated extract was collected with 7 mL of distilled water, and freeze-dried, obtaining *Rosa eglanteria* extract as powder yielding 21.07 mg/mL.

### Preparative example 2: Preparation of Centella asiatica extract

The *Centella asiatica* extract was obtained by freeze-drying the commercially available hydroalcoholic extract comprising 20% of aerial parts of the plant with a 40% Vol. of alcohol, pH of 4.65 and a density of 0.90. Yielding 64.75 mg/mL of powder. Also in the lack of a commercially available extract, the same extraction procedure as with the *Rosa eglanteria* could be performed.

### Preparative example 3: Preparation of the buffer saturated with ZnO

3 mg of ZnO, 1576 mg of trometamol hydrochloride and 8766 mg of NaCl were added to 1 L of distilled water, stirred, and followed by a filtration process with a nylon 0.45 µm filter to eliminate the excess of ZnO (0.1 mg) not dissolved and possible impurities. Then the pH is finally adjusted to 7.4.

### Example 1: Preparation of the liposomal composition

This example discloses the process for preparing the liposomal composition, which was carried out following substantially the disclosure of Vázquez-González et al., Enhanced topical delivery of hyaluronic acid encapsulated in liposomes: A surface-dependent phenomenon, Coll. Surf. B: Biointerfaces, 2015, 134, 31-39.

A chloroform-methanol (2:1, v/v) solution containing L-α-phosphatidylcholine (PC) (egg yolk, 99% purity) was placed in a glass balloon and dried in a rotary evaporator at room temperature protected from light. The resulting thin film was kept under high vacuum overnight to remove any traces of organic solvent. Multilamellar liposomes were obtained by redispersion of the thin film in 10 mM Tris·HCl, 150 mM NaCl buffer, ZnO to saturation, pH 7.4 (Preparative Example 3), buffer that included already the lyophilized extracts of *Rosa eglanteria* and *Centella asiatica* already dissolved therein.

This redispersion was performed by rising the temperature of the mixture over the Transition temperature (Tm) of the lipids and then shook vigorously during 10 s with a Vortex followed by 10 s of sonication on a standard sonic bath. After this, the flask was placed on ice to lower the temperature of the mixture and obtain temperatures under the Tm. This cycle was performed 5 times in order to increase the encapsulation rate and obtain stable multilamellar liposomes.

5 mL of the composition comprised: 100 mg of freeze-dried hydroalcoholic extracts (50 mg of *Rosa eglanteria* (Preparative Example 1), 50 mg of *Centella asiatica* (Preparative Example 2), 50 mg L-α-phosphatidylcholine, 14.5 µg of ZnO, 7.85 mg of trometamol hydrochloride and 43.8 mg of NaCl.

The content in the liposomes of the extract of *Rosa eglanteria* was 1.43 mg/mg PC and of the extract of *Centella asiatica* was 0.25 mg/mg PC.

Thus, the composition comprised 84 wt% of the extracts encapsulated by liposomes, providing the sustained release of the actives, and 16 wt% of the extracts were non-encapsulated providing the boost effect of the composition.

It was measured the polydispersity index of the liposomal composition which indicates the broadness of the particle size distribution measured by the diffraction of the liposomes by means of a laser (Stetefeld et al., Dynamic light scattering: a practical guide and applications in biomedical sciences, Biophys. Rev., 2016, 8, 409-427.) both in absence and in presence of ZnO. The presence of ZnO reduced surprisingly the polydispersity of the liposomal composition from 0.697 to 0.273 meaning a more stable and regular sizing of the generated liposomes.

### Example 2: Preparation of the liposomal composition

This example discloses the process for preparing the liposomal composition, wherein the liposomes of each extract are prepared separately.

### 2.a.- Preparation of liposomes containing Rosa eglanteria freeze dried extract

A chloroform-methanol (2:1, v/v) solution containing L-α-phosphatidylcholine (PC) (egg yolk, 99% purity) was placed in a glass balloon and dried in a rotary evaporator at room temperature protected from light. The resulting thin film was kept under high vacuum overnight to remove any traces of organic solvent. Multilamellar liposomes were obtained by redispersion of the thin film in 10 mM Tris·HCl, 150 mM NaCl buffer, ZnO to saturation, pH 7.4 (Preparative Example 3), buffer that included already the lyophilized extract of Rosa eglanteria already dissolved therein.

This redispersion was performed by rising the temperature of the mixture over the Transition temperature (Tm) of the lipids and then shook vigorously during 10 s with a Vortex followed by 10 s of sonication on a standard sonic bath. After this, the flask was placed on ice to lower the temperature of the mixture and obtain temperatures under the Tm. This cycle was performed 5 times in order to increase the encapsulation rate and obtain stable multilamellar liposomes.

5 mL of the composition comprised: 100 mg of freeze-dried hydroalcoholic extract of *Rosa eglanteria* (Preparative Example 1), 50 mg L-α-phosphatidylcholine, 14.5 µg of ZnO, 7.85 mg of trometamol hydrochloride and 43.8 mg of NaCl.

The content in the liposomes of the extract of *Rosa eglanteria* was 1.43 mg/mg PC.

Thus, the composition comprised 71.5 wt% of the *Rosa eglanteria* freeze-dried extract encapsulated by liposomes, providing the sustained release of the actives, and 28.5 wt% of the extract were non-encapsulated providing the boost effect of the composition.

### 2.b.- Preparation of liposomes containing Centella asiatica freeze dried extract

A chloroform-methanol (2:1, v/v) solution containing L-α-phosphatidylcholine (PC) (egg yolk, 99% purity) was placed in a glass balloon and dried in a rotary evaporator at room temperature protected from light. The resulting thin film was kept under high vacuum overnight to remove any traces of organic solvent. Multilamellar liposomes were obtained by redispersion of the thin film in 10 mM Tris·HCl, 150 mM NaCl buffer, ZnO to saturation, pH 7.4 (Preparative Example 3), buffer that included already the lyophilized extract of Centella asiatica already dissolved therein.

This redispersion was performed by rising the temperature of the mixture over the Transition temperature (Tm) of the lipids and then shook vigorously during 10 s with a Vortex followed by 10 s of sonication on a standard sonic bath. After this, the flask was placed on ice to lower the temperature of the mixture and obtain temperatures under the Tm. This cycle was performed 5 times in order to increase the encapsulation rate and obtain stable multilamellar liposomes.

5 mL of the composition comprised: 100 mg of freeze-dried hydroalcoholic extract of *Centella asiatica* (Preparative Example 2), 50 mg L-α-phosphatidylcholine, 14.5 µg of ZnO, 7.85 mg of trometamol hydrochloride and 43.8 mg of NaCl.

The content in the liposomes of the extract of *Centella asiatica* was 0.25 mg/mg PC.

Thus, the composition comprised 12.5 wt% of the *Centella asiatica* freeze-dried extract encapsulated by liposomes, providing the sustained release of the actives, and 87.5 wt% of the extract were non-encapsulated providing the boost effect of the composition.

Once both single extract compositions were prepared, three different actions were done: mixing both formulations to be used for preparing the pharmaceutical composition; mixing both formulations and freeze-dry to use for the pharmaceutical composition, or freeze-drying both compositions independently and mixing the resulting powder to use for the pharmaceutical composition.

### Example 3: Preparation of the liposomal composition in freeze-dried form

The liposomal composition obtained in Example 1 comprising: 100 mg of freeze-dried hydroalcoholic extracts (50 mg of *Rosa eglanteria* (Preparative Example 1), 50 mg of *Centella asiatica* (Preparative Example 2), 50 mg L-α-phosphatidylcholine, 14.5 µg of ZnO, 7.85 mg of trometamol hydrochloride and 43.8 mg of NaCl, per 5 mL, was freeze-dried to obtain the liposomal composition in freeze-dried form.

Further batches were freeze-dried adding a 5 wt% (w/w) of lactose or cellobiose as cryoprotectants.

### Example 4: Preparation of the liposomal composition of the Rosa eglanteria extract in freeze-dried form

The liposomal composition obtained in Example 2a comprising 50 mg of freeze-dried hydroalcoholic extract of *Rosa eglanteria* (Preparative Example 1), 25 mg L-α-phosphatidylcholine, 7.25 µg of ZnO, 3.93 mg of trometamol hydrochloride and 21.9 mg of NaCl per 5 mL, was freeze-dried to obtain the liposomal composition of the *Rosa eglanteria* extract in freeze-dried form.

Further batches were freeze-dried adding a 5 wt% (w/w) of lactose or cellobiose as cryoprotectants.

### Example 5: Preparation of the liposomal composition of the Centella asiatica extract in freeze-dried form

The liposomal composition obtained in Example 2b comprising 50 mg of freeze-dried hydroalcoholic extract of *Centella asiatica* (Preparative Example 2), 25 mg L-α-phosphatidylcholine, 7.25 µg of ZnO, 3.93 mg of trometamol hydrochloride and 21.9 mg of NaCl, per 5 mL, was freeze-dried to obtain the liposomal composition of the *Centella asiatica* extract in freeze-dried form.

Further batches were freeze-dried adding a 5 wt% (w/w) of lactose or cellobiose as cryoprotectants.

### Example 6: Preparation of an extemporaneous spray

In this example it is disclosed the preparation of an extemporaneous spray comprising the liposomal composition of the invention.

On a two compartment spray bottle for powder and liquid, that contains on one side 5 mL of distilled water and on the other the equivalent of 5 mL of the freeze-dried liposomes.

The user breaks the seal mixing both compartments and after vigorous mixing it is ready to be applied.

### Example 7: Preparation of extemporaneous drops

This example discloses the preparation of extemporaneous drops comprising the liposomal composition of the invention.

On a two compartment drops bottle for powder and liquid, that contains on one side 2.5 mL of distilled water and on the other the equivalent of 5 mL of the freeze-dried liposomes.

The user breaks the seal mixing both compartments and after vigorous mixing it is ready to be applied.

### Example 8: Preparation of a gel

In this example it is disclosed the preparation of a gel comprising the liposomal composition of the invention.

### a) preparation from the liposomal formulation

CMC was slowly added to the liposomal formulation employing an Ultra-Turrax homogenizer.

### b) preparation from the freeze-dried liposomal formulation

CMC was predissolved in buffer (half of the total volume to be prepared) employing an Ultra-Turrax homogenizer. The rest of the volume was added containing the freeze-dried liposomal formulation, which was previously reconstituted in distilled water before mixing them with the CMC predissolved portion taking in account that the amount of freeze-dried powder needed to match the total volume to be produced in order to maintain the same concentrations as for the preparation from the liposomal formulation.

### Example 9: Preparation of an hydrocolloid patch

This example discloses the preparation of an hydrocolloid patch comprising the liposomal composition of the invention.

CMC was predissolved in buffer (half of the total volume to be prepared) employing an Ultra-Turrax homogenizer. The rest of the volume was added including the liposomal formulation and the glycerol.

The liposomal formulation could be added "as-is" without being freeze-dried or if the freeze-dried liposomes were used these needed to be reconstituted in distilled water before mixing them with the CMC predissolved portion.

The mixture was homogenized by gently mixing and then poured on rectangular molds to be dried on an oven at 45 °C for 24 h.

The generated films were kept on a desiccator for 3 days and placed on a proper patch to be easily applied.

### Example 10: Permeation of the liposomal composition

This example discloses the permeation *ex-vivo* in human skin using Franz cells of vertical diffusion in the presence of a lipophilic medium (Transcutol^{®}, diethylene glycol ethyl ether) and in the presence of an aqueous buffer as hydrophilic receiving media.

The experiments were performed under sink conditions, comprising 5 to 10 times more volume of solution than the saturation volume of the active extracts, in order to prevent the solubility thereof from interfering during *ex-vivo* release. To describe the kinetics of drug release from liposomes, the results were fitted to different mathematical models, as disclosed in Vázquez-González et al., Improving ex vivo skin permeation of non-steroidal anti-inflammatory drugs: Enhancing extemporaneous transformation of liposomes into planar lipid bilayers, Int. J. Pharm., 2014, 461(1-2), 427-36.

Data were analysed using GraphPadprism 3.0 (Graphpad Software Inc., California, EUA) and the professional editor WinNonlin^{®} 3.3 (Pharsight Corporation, Mountain View, CA).

The formulation that was accumulated in the stratum corneum, after the *ex-vivo* permeation test on human skin, was removed with a 0.05% sodium lauryl sulfate solution, washed with distilled water and dried with filter paper. The impregnated area of the human skin was trimmed and weighed, and the active extracts content was extracted with an ethanol-water solution (1:1, v/v) using an ultrasonic probe. The amount of resulting active extracts in the solution was analysed using HPLC.

The active extracts were analysed by HPLC (HP1100, Chemstations, Agilent Technologies, USA). Samples were injected into a C18 reversed-phase column (spherisorb ods2 5µ 15x0.46 cm). The wavelength in the UV-Vis detector was set at 279 nm for *Rosa eglanteria* extract and 324 nm for *Centella asiatica* extract from the absorption spectra of the extracts (Figure 1) obtained by spectrophotometry (UV-2410PC, Shimadzu, JP). The method used a mobile phase composed 40/50 by H₂O + 0.1% Orthophosphoric Acid/Acetonitrile. The flow rate was 1 mL/min and the analysis time per sample was 3 minutes.

Figure 2A shows the results of permeation studies of free active extracts in the solution and in the presence of liposomes, when the receiving medium is Transcutol^{®}. It was observed that the concentration of *Rosa eglanteria* extract in the skin after 24 hours increases by 75% when said extract is encapsulated within liposomes compared to the value when it was free in the medium. The same study with *Centella asiatica* extract showed no significant differences between the free molecule and when it is encapsulated within liposomes. In order to further analyse the effect of *Centella asiatica,* the experiments were repeated using a buffer solution as a receiving medium instead of Transcutol^{®} (Figure 2B). In this case, the values obtained with *Rosa eglanteria* extract were very similar to those obtained from Transcutol^{®}, but the values of retained *Centella asiatica* extract in the skin were increased, obtaining values approximately 40% higher when the molecule was in the presence of liposomes than when it was free in the medium. These results are indicative that the active molecules analysed present in *Centella asiatica* do not have much affinity for lipophilic phases and that they prefer to diffuse towards more polar regions.

## Claims

1. A liposomal composition comprising:
a) liposomes,
b) a *Rosa eglanteria* extract,
c) a *Centella asiatica* extract,
d) zinc oxide, and
e) a buffer,
wherein at least 50 wt%, preferably at least 55 wt% of said extracts are encapsulated in the liposomes.

2. The liposomal composition according to claim 1, wherein it comprises liposomes, which comprise phosphatidylcholine.

3. The liposomal composition according to claims 1 or 2, wherein the content of phosphatidylcholine in the liposomal composition is comprised between 5 mg and 15 mg per mL of the liposomal composition.

4. The liposomal composition according to claim 2, wherein it comprises liposomes, which comprise a combination of phosphatidylcholine and cholesterol.

5. The liposomal composition according to claim 4, wherein the content of phosphatidylcholine in the liposomal composition of the invention is comprised between 5 mg and 15 mg per mL of the liposomal composition, and the content of cholesterol is comprised between 0.1 mg and 15 mg per mL of the liposomal composition.

6. The liposomal composition according to any one of claims 1 to 5, wherein the liposomal composition comprises the liposomes, which comprise between 75 wt% and 95 wt% of *Rosa eglanteria* extract and 5 wt% and 25 wt% of *Centella asiatica* extract on the total extract content encapsulated in the liposomes.

7. The liposomal composition according to any one of claims 1 to 6, wherein the *Rosa eglanteria* and the *Centella asiatica* extracts are hydroalcoholic extracts.

8. The liposomal composition according to claim 7, wherein the hydroalcoholic extracts are freeze dried.

9. The liposomal composition according to any one of claims 1 to 8, wherein the buffer comprises Tris-HCl and sodium chloride, NaCl, adjusted at a pH of about 7.4.

10. A process for preparing the liposomal composition of any one of claims 1 to 9, wherein it comprises:
1) dissolving the lipid in an organic solvent, in a vessel,
2) evaporating the solvent of the solution obtained in step 1) to yield a film of the lipid on the walls of the vessel,
3) redispersing the film obtained in step 2) with a buffer containing the extract of *Rosa eglanteria* and/or the extract of *Centella asiatica,* and zinc oxide at saturation, and
4) sonicating the dispersion obtained in step 3) to form liposomes.

11. The process according to claim 10, wherein the liposomal composition is freeze-dried.

12. A pharmaceutical composition, which comprises the liposomal composition according to any one of claims 1 to 9.

13. The pharmaceutical composition according to claim 12, wherein the form thereof is selected from extemporaneous spray, extemporaneous drops, gel and hydrocolloid patch.

14. The liposomal composition according to any one of claims 1 to 9 or the pharmaceutical composition according to any one of claims 12 or 13 for medical use, preferably for use in the treatment of wounds and scars.

15. The cosmetic use of liposomal composition according to any one of claims 1 to 9 or the pharmaceutical composition according to any one of claims 12 or 13 for the treatment of wounds and scars.
